# DEMANDE DE BREVET EUROPEEN

(11) **EP 0 677 287 A2**
(43) Date de publication de la demande: **18.10.1995**
(21) Numéro de dépôt: 95400399.2
(22) Date de dépôt: 27.02.1995
(51) Int. Cl.: A61K 7/42, A61K 7/48

(54) **Composition cosmétique pour la protection solaire**

(30) Priorité: 28.02.1994 FR 9402372
(71) Demandeur: ROUSSEL UCLAF, F-93230 Romainville (FR)
(72) Inventeur: Bobier-Rival, Carinne, Port-Cergy, F-95000 Cergy (FR); Bourgade, Sylvie, F-78500 Sartouville (FR); Fructus, Alain, F-92400 Courbevoie (FR)
(74) Mandataire: Vieillefosse, Jean-Claude

(57) **Abrégé**

L'invention a pour objet les compositions cosmétiques comprenant des phases huileuses et aqueuses et un ou plusieurs protecteurs solaires et d'autres additifs, qui contiennent de l'huile de silicone, une phase aqueuse et de l'oxyde de titane micronisé et éventuellement un produit anti-inflammatoire.

## Description

La présente invention concerne des compositions cosmétiques comprenant des particules de dioxyde de titane pour limiter les effets du rayonnement solaire.

La protection de la peau contre les coups de soleil et autres lésions associées avec l'exposition aux radiations ultra-violet, est d'une grande importance, puisqu'un nombre de personnes plus grand que jamais s'adonne aux activités de loisirs à l'extérieur en s'exposant de plus en plus à la lumière du soleil.

Depuis longtemps l'industrie cosmétique met à la disposition des personnes, des produits tels que des gels, des crèmes, des huiles incorporant des filtres solaires et des réflecteurs solaires. Ce sont des substances chimiques, organiques ou inorganiques qui absorbent ou reflètent les rayonnements UVA et/ou B.

Il est en effet intéressant de limiter les effets du rayonnement solaire au niveau cutané, en particulier ceux des UVB et A. Les premiers provoquent l'érythème solaire et à plus long terme des altérations cutanées bénignes et malignes, les seconds sont le principal facteur du vieillissement cutané.

Les produits protecteurs solaires sont d'une très grande utilité car ils apportent réellement une protection de la peau qui est mesurée par ce que l'on appelle un facteur de protection.

Cependant leur présence sur la peau, si elle est très bénéfique par la protection contre les radiations UVA et B, n'est pas complètement inoffensive.

Il apparaissait donc judicieux de trouver de nouvelles compositions cosmétiques qui limitent les effets du rayonnement sans qu'elles soient offensives pour la peau.

Dans l'art antérieur, notamment dans le brevet FR-B 2615860 et dans la demande de brevet EP-A 509904, sont décrites des dispersions huileuses et crèmes solaires, mais on n'obtient pas de composition avec des facteurs de protection satisfaisants.

La présente invention a pour objet une composition cosmétique comprenant des phases huileuses et aqueuses et un ou plusieurs protecteurs solaires et éventuellement d'autres additifs, composition cosmétique qui contient de l'huile de silicone, une phase aqueuse et de l'oxyde de titane micronisé. Cette composition donne une excellente protection solaire sans utiliser trop de réflecteurs de soleil. De plus, la protection reste efficace, même après plusieurs bains (par exemple dans la mer).

Cette composition cosmétique peut contenir aussi un produit anti-inflammatoire, préférentiellement des glycoprotéines extraites d'une souche microbienne d'Hafnia.

Préférentiellement, la composition cosmétique contient 2 à 15 %, spécialement 3 à 10 % du poids total de la composition, d'huile de silicone. On utilise préférentiellement des silicones volatils comme par exemple le Dow Corning® 344 ou 345 avec un composé tensio actif. On peut utiliser une dispersion d'un tensio-actif silicone de haut poids moléculaire dans un silicone volatil du type cyclométhicone (par exemple le Dow Corning® Q2 3225 C).

La composition cosmétique contient 20 à 70 % du poids total de la composition, d'eau et 2 à 25 % du poids total de la composition, d'oxyde de titane micronisé.

Comme oxyde de titane, on utilise préférentiellement le dioxyde de titane sous forme de particules fines pratiquement sphériques, qui ont une dimension moyenne de 10 à 50 nm, préférentiellement de 20 à 30 nm. Les particules de dioxyde de titane à utiliser pour les compositions de la présente invention peuvent contenir aussi des oxydes ou oxydes hydratates, par exemple d'aluminium, de fer ou de silicium, mais la teneur du dioxide de titane est préférentiellement supérieure à 96 %, spécialement 96,5 %. Le dioxyde de titane très fin peut être obtenu par pyrogénation du chlorure de titane. Puis on applique un traitement en surface au moyen du silane d'autres produits pour rendre le dioxyde de titane hydrophobe.

En particulier, la composition contient 5 à 15 % du poids total de la composition,d'oxyde de titane pyrogéné traité en surface avec du silane ou non traité et pré-dispersé.

Comme oxyde de titane, on peut mentionner les produits Degussa® P25, T805 et T202 parmi lesquels le T805 est 5 préféré.

On peut également utiliser de l'oxyde de titane pyrogéné traité ou non traité en surface et prédispersé dans un liquide convenable comme un polyol, un hydrocarbure, un ester gras, un silicone ou un triglycéride. Parmi les polyols, on peut citer particulièrement le propylène glycol, parmi les hydrocarbures le polyisobutène ou l'huile de vaseline, parmi les esters l'octyldodécyl néopentanoate ou le benzoate d'alcool C12-C14, parmi les silicones, un silicone volatil et parmi les triglycérides, le caprylique-caprique.

Par exemple dans le cas d'un oxyde de titane pyrogéné non traité en surface, on peut faciliter sa dispersion dans un polyisobutène en ajoutant un agent dispersant comme un ester de phosphate. On peut particulièrement employer le PPG-5 Ceteth-10 Phosphate.

On peut également employer un oxyde de titane micronisé non pyrogéné. On peut par exemple, employer le produit de la Société KEMIRA dénommé M 160, dont les cristaux ont une taille d'environ 17 nanomètres et qui est traité en surface avec un mélange d'alumine et d'acide stéarique.

On peut également employer un oxyde de titane micronisé non pyrogéné comme celui de la Société TIOXIDE dénommé TIOVEIL MOTG et qui est une prédispersion d'oxyde de titane micronisé dispersé dans un mélange d'huile minérale et de triglycéride.

En particulier, la composition contient 5 à 10% du poids total de la composition, d'oxyde de titane micronisé non pyrogéné traité en surface ou prédispersé.

La composition cosmétique contient préférentiellement 0,001 à 4 % du poids total de la composition, d'un composé à activité anti-inflammatoire choisi par exemple parmi les biolysats Hafnia, acide 18β glycyrrhétinique ou enoxolone et ses dérivés, glycyrrhétinate d'ammonium, stéaryl glycyrrhétinate, et son complexe avec les phospholipides (phytosomes (R)), l'α bisabolol, l'alcool batylique, l'extrait d'hirudine, l'extrait de meristem, l'extrait d'ougon, l'extrait de mimosa tenuiflora, l'extrait de karité, le panthénol, l'acide ximménique, l'huile de maïs peroxydée.

L'ajout d'un tel composé peut notamment, diminuer l'état inflammatoire de la peau.

Parmi ces composés à activité anti-inflammatoire, on peut citer de façon préférentielle, les biolysats d'Hafnia, et l'invention a notamment pour objet une composition contenant 0,001 à 1 % du poids total de la composition, de glycoprotéïnes extraites d'une souche microbienne d'Hafnia.

Le brevet FR-B 84 06559 décrit notamment de tels biolysats Hafnia.

Les compositions cosmétiques selon l'invention peuvent contenir aussi d'autres additifs, comme d'autres huiles, des parfums, des agents dispersants, des émulsifiants, d'autres filtres solaires ou des conservateurs. On préfère préparer une composition qui ne contient ni filtres solaires ni parfums supplémentaires.

Comme émulsifiants on peut citer par exemple les produits suivants (avec le pourcentage préféré) :
- Arlacel 83® de ICI (Sorbitan sesquioleate) 0,5 à 3 %
- Isolan GI 34® de Goldschmidt (Polyglycéryl-3 isostearate) 0,5 à 4 %
- Arlacel 989® de ICI (PEG-7 Hydrogenated Castor Oil) 1 à 5%
- INWITOR 780 K® de Hüls (glyceryl isostearate) 0,2 à 4 %
- Arlacel 1689® de ICI (Sorbitan oleate/polyglycéryl-3 ricinoléate) 0,5 à 6 %
- Grillocose IS® de Grillo (méthylglucose dioleate) 0,5 à 4 %
- Grillocose DO® de Grillo (méthylglucose dioleate) 0,5 à 3 %
- ELFACOS ST 9® de AKZO (PEG-45/dodécyl glycol copolymer) 0,2 à 2 %

L'huile pour la composition cosmétique peut être n'importe quelle huile dont la présence est souhaitable dans la dispersion résultante. Ces huiles sont ordinairement les huiles végétales comme l'huile de jojoba, d'olive, d'avocat, de macadamia, des huiles minérales comme l'huile de vaseline, des hydrocarbures comme le dioctyl cyclohexane ou l'isohexadécane, des esters comme le palmitate de cétyle ou l'isocétyle, des cires naturelles comme la cire d'abeille, de carnauba, de candellila, des triglycérides semi synthétiques comme les triglycérides capryliques ou capriques, des alcools gras comme l'alcool cétylique ou stéarylique, des esters du propylène glycol comme le monostéarate de propylène glycol, des esters de l'acide benzoïque comme le FINSOL TN, les diesters d'acides gras saturés, par exemple le 12-stéaroylstéarate de 2-octyl-dodécyle, l'alcool oléylique et le palmitate d'isopropyle, le tétracaprylate/caprate de pentaérythritol, les diesters de propylèneglycol d'acides gras de noix de coco et le tétraisostéarate de pentaérythritol.

Les compositions peuvent contenir un agent dispersant organique pour favoriser la dispersion du dioxyde de titane particulaire dans l'huile de silicone et les autres huiles choisies. De nombreux types d'agents dispersants organiques ont été mis au point et sont disponibles pour faciliter la dispersion des particules dans des milieux huileux, par exemple des acides carboxyliques substitués, des bases de savons et des polyhydroxyacides. De manière typique, l'agent dispersant peut être un composé de formule X-CO-AR dans laquelle A est un groupe de pontage divalent, R est un groupe amino primaire, secondaire ou tertiaire ou son sel avec un acide ou un groupe salin d'ammonium quaternaire et X est le résidu d'une chaîne de polyester qui, avec le groupe -CO-, dérive d'un acide hydroxycarboxylique de formule HO-R'-COOH. Des exemples d'agents dispersants typiques sont ceux à base d'acide ricinoléique, d'acide hydroxystéarique, d'acide gras d'huile de ricin hydrogénée qui contient en plus de l'acide 12-hydroxystéarique de faibles quantités d'acide stéarique et d'acide palmitique.

On peut aussi utiliser des agents dispersants à base d'un ou plusieurs polyesters ou sels d'un acide hydroxycarboxylique et d'un acide carboxylique exempt de groupes hydroxy. On peut utiliser des composés de divers poids moléculaires.

D'autres agents dispersants appropriés sont les mono-esters d'alcanolamides d'acides gras et d'acides carboxyliques et leurs sels à base d'acides gras saturés ou insaturés en C₆-C₂₆. Les alcanolamides sont par exemple à base d'éthanolamine, de propanolamine ou d'aminoéthyléthanolamine.

D'autres agents dispersants sont ceux à base de polymères ou de copolymères d'acide acrylique ou méthacrylique, par exemple des copolymères séquencés de ces monomères.

D'autres agents dispersants de forme générale semblable sont ceux ayant des groupes éthylèneoxy dans les radicaux qui les constituent comme ceux à base des phosphates esters oxyéthylénés.

Comme conservateurs on peut utiliser par exemple le i méthylparaben, le propylparaben, le O-cymen-5-ol ou le 2-bromo 2-nitropropane 1,3-diol.

L'invention concerne aussi un procédé de préparation d'une composition cosmétique telle que définie ci-dessus, caractérisé en ce qu'on prépare une émulsion avec une phase huileuse comprenant l'huile de silicone et l'oxyde de titane micronisé et avec une phase aqueuse, et qu'on ajoute éventuellement à cette émulsion d'autres additifs.

Le procédé de préparation dépend du type de composition.

Enfin des réflecteurs solaires autres que l'oxyde de titane micronisé peuvent être ajoutés à ces compositions cosmétiques pour en renforcer le pouvoir protecteur.

Ce sont généralement des produits insolubles dans les phases huileuses et aqueuses et ils constituent donc une phase supplémentaire. Ils sont choisis par exemple parmi les produits suivants :
- les perfluoroéthers (comme les FOMBLIN® de la Sté Montecotini),
- les pigments insolubles comme :
   . les oxydes de titane,
   . oxyde de titane rutile,
   . les oxydes de titane non micronisés,
   . un deuxième oxyde de titane traité en surface (par des silicones ou par des acides aminés, ou par de la lécithine, ou par des stéarates métalliques),
   . oxyde de fer,
   . oxyde de fer traité en surface par des silicones, ou par de la lécithine, ou par des stéarates métalliques,
   . oxyde de zinc,
   . oxyde de zinc micronisé (comme l'UFZO® de Cosmo Trends Japon Corporation ou le Z-cote de la Société SUNSMART),
   . mica recouvert d'oxyde de titane.

La présente invention a également pour objet l'application à titre de produit cosmétique des compositions telles que définies ci-dessus.

La présente invention a aussi pour objet une méthode de traitement cosmétique caractérisée en ce que l'on utilise les compositions cosmétiques telles que définies ci-dessus. Dans la méthode de traitement cosmétique utilisant les compositions de l'invention, lesdites compositions sont utilisées par application cutanée comme les compositions usuelles.

La présente invention a aussi pour objet à titre de médicament, des compositions telles que définies ci-dessus.

### EXEMPLES

### 1 - Préparation des compositions cosmétiques

A) On mélange à température ambiante (entre 20° et 60°C) les substances suivantes :
   - huile de silicone (Dow Corning® Q2 3225C) 6,00 g
   - PEG-7 hydrogenated castor oil (ARLACEL® 989) 2,00 g
   - Huile de silicone volutile (cyclométhicone) 2,00 g
   - Stéarate d'isocétyle 3,00 g
   - PEG-45/Dodécyl glycolcopolymer (ELFALOS ST9®) 2,00 g
   - PPG-3 Myristyl éther 2,00 g
   - C12-15 Alcools benzoates (FINSOLV® TN) 2,00 g
   - Tocophérol 0,05 g
   - Oxyde de titane pyrogéné traité par du silane en surface, dispersé dans du polyisobutène hydrogéné dans la proportion respective de 38 % d'oxyde de titane et 62 % du polyisobutène 30,00 g

   On agite modérément jusqu'à ce que l'oxyde de titane soit bien dispersé. Ceci constitue la phase grasse.
B) On prépare ensuite la phase aqueuse de la façon suivante, on dissout dans 4 g de polyéthylène glycol 400 (PEG.400), 0,25 g de méthylparaben, 0,15 g de propylparaben et 0,1 g de 0-cymen-5-ol ; on dissout dans 46 g d'eau filtrée et déminéralisée, 0,25 g de sulfate de magnésium, 0,05 g de bromo-nitropropane-diole (Bromopol), 0,05 g de sel de l'acide éthylène diamine tétraacétique ;
   on ajoute à cette solution, le mélange des conservateurs dans le PEG 400 (humectant pour la solution des conservateurs).
C) Cette phase aqueuse est ensuite versée lentement sans agitation vigoureuse dans la phase grasse. L'émulsion eau/huile (E/H) se forme et on agite encore 30 minutes.
D) Pour préparer une composition contenant un produit anti-inflammatoire, on dissout ensuite 0,02 g de Biolysat Hafnia dans le complément d'eau à 100 g. Puis on ajoute lentement cette solution à l'émulsion précédente en agitant modérément, cela constitue l'émulsion E/H complète. La composition finale contient 11,4 % en poids de dioxyde de titane.

La protection de la peau contre les coups de soleil avec cette préparation (I) est décrite ci-après.

### EXEMPLE 2 :

comme décrit dans l'exemple 1, on prépare une composition dont la phase grasse est ainsi constituée :
- ABIL EM 90® (polysiloxane ; Goldschmidt) 4,0 g
- Cyclomethicone 4,0 g
- FINSOLV TN® 1,0 g
- Tocophérol 0,05 g
- Oxyde de titane micronisé traité par du silane en surface 8,0 g
- Polyisobutène hydrogéné 22,0 g

La phase grasse est bien homogénéisée.
La phase aqueuse est composée de :
- Eau 57,5 g
- EDTA 0,05 g
- Sulfate de magnésium 0,8 g
- PEG 400 1,5 g
- Méthyl paraben 0,25 g
- Propyl paraben 0,15 g
- O-Cymen-5-OL 0,1 g
- Bromopol 0,05 g
- Biolysat Hafnia 0,02 g
- Parfum 0,5 g

La phase aqueuse est mélangée à la phase grasse sous agitation vigoureuse à température ambiante pendant 20 minutes. L'émulsion se forme rapidement. Elle est fine et régulière et contient 8 % d'oxyde de titane.

### EXEMPLE 3 :

On prépare en analogie à l'exemple 1, la phase grasse suivante :
- huile de silicone (DC Q2 3225 C) 9,0 g
- Cyclomethicone 8,0 g
- Stéarate d'isocétyl 3,0 g
- PPG-3 myristyl éther 2,0 g
- ELFACOS ST 9 2,0 g
- Propyl paraben 0,15 g
- Tioveil® OP 10,0 g
- Parfum 0,5 g

La phase aqueuse est la suivante :
- Eau 61,3 g
- Sulfate de magnésium 0,7 g
- Méthyl paraben 0,25 g
- PEG 400 4,0 g
- O-Cymen-5-OL 0,1 g

Le mode opératoire est le même que dans l'exemple 2.

### EXEMPLE 4 : Composition cosmetique de comparaison de type Huile/eau sans émulsionnant (II à IV)

On prépare des compositions avec 5,18/10,26 et 15,4 % du poids total de la composition de l'oxyde de titane. Phase grasse :
- Huile de vaseline 8,0 g
- Huile de ricin 6,0 g
- Huile de jojoba 4,0 g
- Céramides HO3® 0,1 g
- Oxyde de titane micronisé traité par du silane en surface 5,18 g
   (ou 10,26 g ou 15,4 g)
- Polyisobutène hydrogéné 8,32 g
   (ou 16,74 g ou 25,1 g)
- Diméthicone 2,0 g
- Acétate de tocophérol 1,0 g

Phase aqueuse :
- Eau 51,37 g
   (ou 35,87 g ou 24,37 g)
- PECOGEL S 1120® 3,0 g
- LUBRAGEL OIL® 3,0 g
- Glycérine 3,0 g
- PEMULEN TR2® 0,3 g
- Amercell HM 1500 0,1 g
- Méthyl paraben 0,25 g
- Propyl paraben 0,15 g
- Biosol 0,1 g
- PEG 400 4,0 g
- EDTA 0,05 g
- Soude pure 0,05 g
- Biolysat Hafnia 0,03 g

La phase aqueuse est mélangée à la phase grasse à 80°C pendant 30 minutes. Puis on refroidit lentement sous agitation modérée. Le biolysat Hafnia est introduit à température ambiante.
Comme indice de protection moyen, on trouve pour les 3 préparations : 5,5/9,1 et 17,5.

### EXEMPLE 5 :

On prépare trois préparations de comparaison du type Eau/huile sans silicone (V, VI et VII) qui contiennent 5 %, 10 % ou 15 % de TiO₂.
Phase grasse :
- Huile de vaseline 5,0 g
- Arlacel 582® 5,0 g
- Arlacel 989® 1,5 g
- Arlamol S7 5,0 g
- Huile de jojoba 5,0 g
- Céramides HO3® 0,1 g
- Oxyde de titane pyrogéné traité par du silane en surface 5,0 g
   (ou 10,0 g ou 15,0 g)
- Polyisobutène hydrogéné 8,5 g
   (ou 17,0 g ou 25,5 g)

Phase aqueuse :
- Eau 60,3 g
   (ou 46,8 g ou 33,3 g)
- Méthyl paraben 0,25 g
- Propyl paraben 0,15 g
- Biosol 0,1 g
- PEG 400 4,0 g
- Sulfate de magnésium 0,07 g
- Biolysat Hafnia 0,03 g

La phase aqueuse est versée dans la phase grasse à 80°C en agitant 20 min. Puis on refroidit lentement sous agitation modérée. Le biolysat Hafnia est introduit à température ambiante. On trouve pour les trois préparations un indice de protection moyen de respectivement 4,6 - 8,6 - 18,4.

### Mesure de l'indice de protection solaire

### Source de radiations

Elle est constituée par un simulateur solaire (Dermolum Um ; Müller GmbH Elektronik Optik - Am Bleichbach 7 - 8059 Moosinning - Allemagne).

La partie active de celui-ci est constituée d'une lampe au xénon (Hanovia) d'une puissance de 1.000 watts et d'une lampe à halogénures métalliques (Ultramed Osram) de 1.000 watts.

Les irradiations ont été réalisées par l'intermédiaire d'un sensitomètre. Ce dispositif, constitué d'une plaque comportant neuf orifices circulaires de 15 mm de diamètre et distants de 10 mm les uns des autres, a été mis au contact des différents sites cutanés. L'obturation des orifices, programmée par ordinateur, a permis d'administrer les neuf doses croissantes de radications U.V. (B+A).

### Dispositif d'ajustement du spectre

Le spectre d'émission global de l'ensemble de ces deux sources comprend les U.V.C. (longueurs d'onde inférieure à 5 290 nanomètres), les U.V.B. (290-320 nanomètres), les U.V.A. (320-400 nanomètres), le visible et l'infrarouge.

Pour être plus proche du spectre naturel, ce spectre d'émission global a dû être modifié à ses deux extrémités car il diffère de ce dernier par l'existence de deux grandes raies dans l'infrarouge (vers 800 et 900 nanomètres) apportant beaucoup de chaleur et par la présence de longueurs d'onde inférieures à 290 nanomètres (U.V.C.). L'importante émission infrarouge a été atténuée par l'emploi d'un filtre à eau placé à la sortie de la lanterne.

Un filtre optique "cut of" (WG 305 Schott) de 1 mm d'épaisseur a été placé à la sortie du filtre à eau pour éliminer les longueurs d'onde inférieures à 290 nanomètres et permettre de travailler avec les U.V.B., les U.V.A., le visible et les infrarouges.

### Dispositif de mesure des éclairements énergétiques

La mesure des éclairements énergétiques dans les franges spectrales U.V.B. et U.V.A. a été réalisée à l'aide d'un radiomètre (RMX 3W Vilber-Lourmat), par l'intermédiaire de deux cellules photosensibles : l'une d'elles présente une sensibilité optimale à 312 nanomètres (U.V.B.), l'autre à 365 nanomètres (U.V.A.).

### Méthodes cliniques

Le but de cette étude a été de déterminer l'efficacité photoprotectrice de la composition cosmétique selon la méthode F.D.A. (voir "Fédéral Register : Sunscreen drug products for over the counter human drugs : proposed safety, effective and labeling conditions. Washington D.C., Department of Health Education and Welfare FDA, vol. 43 n° 46 p. 38206, 1978").

L'étude a été effectuée en ouvert, non randomisée. La durée de l'étude pour chaque sujet a été de deux jours consécutifs. L'étude a été conduite chez dix adultes volontaires sains dont sept sujets de sexe féminin et trois sujets de sexe masculin. Les sujets ont été considérés comme normaux à la suite d'un examen clinique, ils n'ont reçu aucun médicament, ni cosmétique pendant toute la période d'observation.

Les sujets se sont présentés dans l'unité :
- le premier jour (J1) pour l'application de la préparation à étudier et de la préparation de référence, suivie de l'irradiation sur ces sites traités et sur un site non traité ;
- le deuxième jour (J2) pour la détermination visuelle des Doses Erythémales Minimales (D.E.M.).

### Mode d'application des préparations

La préparation à étudier (voir exemple 1) et la préparation de référence ont été appliquées chacune sur un site de 64 cm² de la partie médiane du dos, à la dose de 2 µl/cm², par massage digital doux (doigt recouvert d'un doigtier), 15 à 20 minutes avant l'exposition.

Les irradiations nécessaires à la recherche des Doses Erythémales Minimales (D.E.M.) sur le site non traité et sur les sites traités respectivement par la préparation à étudier et par la préparation de référence, ont été réalisées à J1 en lumière artificielle, à l'aide d'un simulateur solaire, par administration de neuf doses de radiations U.V. (B+A) (mJ/cm² d'U.V.B.), croissantes en progression géométrique de raison r = 1.25.

La détermination de la D.E.M. sur les sites protégés et sur le site non protégé, a été réalisée 20 heures (±4 heures) après les irradiations (J2).

L'indice de protection individuel de la préparation selon l'invention et de la préparation de référence a été obtenu en effectuant le rapport des D.E.M. après protection à la D.E.M. sans protection.

L'indice de protection moyen de la préparation nouvelle et de la préparation de référence a été obtenu par le calcul de la moyenne arithmétique des indices individuels correspondants.

### Résultats

Avec les dix sujets inclus dans l'étude, les valeurs individuelles des Doses Erythémales Minimales (mJ/cm² d'U.V.B.) sont obtenus sur le site témoin (T : non protégé) et sur le site traité par la préparation de l'exemple 1 selon l'invention (A).

Le tableau montre également les indices de protection individuels (QI) de la préparation nouvelle.

| Sujet | Phototype | D.E.M. | | QI |
|---|---|---|---|---|
| | | T | A | A |
| 1 | III A | 125 | 4443 | 35,54 |
| 2 | II | 195 | 4443 | 22,78 |
| 3 | III A | 156 | 4443 | 28,48 |
| 4 | III A | 100 | 3554 | 35,54 |
| 5 | II | 195 | 4443 | 22,78 |
| 6 | III A | 244 | 6943 | 28,45 |
| 7 | III A | 195 | 4443 | 22,78 |
| 8 | II | 100 | 2274 | 22,74 |
| 9 | III B | 100 | 2274 | 22,74 |
| 10 | III B | 195 | 4443 | 22,78 |

L'indice de protection moyen de la préparation nouvelle est déterminé 26,46 (± 5,32).

L'indice de protection moyen de la préparation de référence est 4,04 (± 0,73).

Aucune réaction d'intolérance locale n'a été observée et aucune sensation désagréable (picotement, démangeaison) d'inconfort ou de gêne n'a été rapportée par les volontaires consécutivement à l'application de la préparation nouvelle.

On voit donc que la préparation de la présente invention présente un indice de protection solaire sur humain en UVB nettement supérieur aux indices de protection des autres types de formules (E/H sans silicone ou H/E sans émulsionnant).

## Revendications

**1)** Composition cosmétique comprenant des phases huileuses et aqueuses et un ou plusieurs protecteurs solaires et d'autres additifs, caractérisée en ce que la composition contient de l'huile de silicone, une phase aqueuse et de l'oxyde de titane micronisé.

**2)** Composition cosmétique selon la revendication 1, caractérisée en ce que la composition contient un produit anti-inflammatoire.

**3)** Composition cosmétique selon la revendication 1 ou 2, caractérisée en ce que la composition contient des glycoprotéines extraites d'une souche microbienne d'Hafnia.

**4)** Composition cosmétique selon une des revendications 1 à 3, caractérisée en ce que la composition contient 2 à 15 % du poids total de la composition, d'huile de silicone, 20 à 70 % du poids total de la composition, d'eau et 2 à 25 % du poids total de la composition, d'oxyde de titane micronisé.

**5)** Composition cosmétique selon une des revendications 1 à 4, caractérisée en ce que la composition contient 5 à 15 % du poids total de la composition, d'oxyde de titane pyrogéné traité en surface avec du silane ou non traité et pré-dispersé.

**6)** Composition cosmétique selon les revendications 1 à 5, caractérisée en ce que la composition contient 5 à 15% du poids total de la composition d'oxyde de titane micronisé non pyrogéné traité en surface ou prédispersé.

**7)** Composition cosmétique selon une des revendications 1 à 6, caractérisée en ce que la composition contient 0,001 à 1 % du poids total de la composition, des glycoprotéines extraites d'une souche microbienne d'Hafnia.

**8)** Procédé de préparation d'une composition cosmétique telle que définie aux revendications 1 à 7, caractérisé en ce qu'on prépare une émulsion avec une phase huileuse comprenant l'huile de silicone et l'oxyde de titane micronisé et avec une phase aqueuse, et qu'on ajoute éventuellement à cette émulsion d'autres additifs.

**9)** Application à titre de produit cosmétique d'une composition telle que définie aux revendications 1 à 7.

**10)** Méthode de traitement cosmétique caractérisée en ce qu'on utilise une composition cosmétique telle que définie aux revendications 1 à 7.

**11)** A titre de médicament, une composition telle que définie aux revendications 1 à 7.
